# EUROPÄISCHE PATENTSCHRIFT

(11) **EP 0 638 296 B1**
(45) Veröffentlichungstag und Bekanntmachung des Hinweises auf die Patenterteilung: **17.11.1999**
(21) Anmeldenummer: 94106516.1
(22) Anmeldetag: 26.04.1994
(51) Int. Cl.: A61C 19/00, A61B 19/00, A47L 15/00, A47L 15/10, B08B 3/02

(54) **Verfahren und Vorrichtung zur Intensivreinigung von ärztlichen, insbesondere zahnärztlichen, Gegenständen**
Method and device for intensive cleaning of medical, especially dental articles
Procédé et dispositif pour le nettoyage intensif d'articles médicaux, notamment dentaires

(30) Priorität: 15.07.1993 DE 4323816
(43) Veröffentlichungstag der Anmeldung: 15.02.1995
(60) Teilanmeldung: 99107442.8
(73) Patentinhaber: Sirona Dental Systems GmbH, 64625 Bensheim (DE)
(72) Erfinder: Hruza, David, Dipl.-Ing., D-88339 Bad Waldsee (DE); Stetter-Alle, Raimund, Dipl.-Ing. (FH), D-89073 Ulm (DE); Trackl, Karl, D-89129 Langenau (DE); Sutter, Ralf, Dipl.-Ing. (FH), D-69469 Weinheim (DE); Beerstecher, Lutz, Dipl.-Ing., D-64625 Bensheim (DE)
(74) Vertreter: Isenbruck, Günter, Dr.

(56) Entgegenhaltungen:
- EP-A- 0 300 945
- EP-A- 0 494 031
- DE-A- 1 403 669
- DE-A- 3 018 872
- DE-A- 3 117 264
- DE-B- 1 185 348
- DE-C- 561 205

## Beschreibung

Die Erfindung bezieht sich auf ein Verfahren sowie eine Vorrichtung zur Intensivreinigung von ärztlichen, insbesondere zahnärztlichen, Instrumenten sowie gegebenenfalls in deren Anwendungsbereich fallenden anderen Gegenständen.

Im medizinischen, insbesondere zahnmedizinischen, Anwendungsbereich müssen die dort eingesetzten Instrumente nach einer Patientenbehandlung einer Intensivreinigung unterzogen werden. Im zahnärztlichen Bereich sind dies Bohr- und Schleifinstrumente (Winkelstücke und Turbinenhandstücke), Spritzhandstücke sowie diverse andere Handinstrumente. Darüber hinaus sind häufig, und dies gilt vornehmlich für den Laborbereich, auch andere Gegenstände, wie Zahnbrücken, Zahnspangen, Gebisse od.dgl. einer solchen Intensivreinigung zu unterziehen.

Aus der DE-A1-30 18 872 ist ein Verfahren zum Reinigen von Gegenständen, insbesondere medizinischen Geräten, bekannt, bei dem Reinigungsflüssigkeit intermittierend auf die Gegenstände gespritzt wird, wobei die Pausen zwischen den Spritzvorgängen so lang sind, daß auf den Gegenständen gebildeter Film der Reinigungsflüssigkeit abgerissen ist, bevor ein weiterer Spritzvorgang beginnt. Das Einspritzen der Reinigungsflüssigkeit erfolgt bei dem bekannten Verfahren mittels Druckluft. Die nach diesem Verfahren arbeitende Vorrichtung ist nach Art einer Geschirrspülmaschine aufgebaut, d.h. die Gegenstände werden einerseits von oben über einen mit Spritzdüsen besetzten Dreharm und andererseits von unten über sog. Langdüsen, die sich in das Innere der zu reinigenden Gegenstände erstrecken, besprüht.

Aus der DE-A1-39 16 446 ist es zu allgemeinen Reinigungszwecken, insbesondere zur Reinigung von Gestein, Bauwerken od.dgl. bekannt, dem Druckwasserstrahl durch Injektorwirkung Luft kontinuierlich oder periodisch regelbar zuzusetzen. Hierzu ist der Druckwasserdüse am Druckwasseraustritt eine Mischkammer mit diversen Lufteintrittsöffnungen vorgelagert, über die Luft angesaugt wird.

In DE-A1-31 17 264 ist eine Kassette zur Ablage und Aufbewahrung von zahnärztlichen Instrumenten beschrieben, welche zwei im wesentlichen waagerecht angeordnete, zylindrische Ständer enthält, die hintereinander mehrere Aufnahmeeinrichtungen für die Instrumente aufweisen. Die Ständer sind schwenkbar gelagert, so daß die aufgesteckten Instrumente einerseits zur Entnahme dargereicht, andererseits zur Ablage und Aufbewahrung vor, wahrend und nach einer Sterilisation benutzt werden können. Aus dem Dokument ergeben sich keinerlei Hinweise auf eine Behandlung der Außenflächen der Instrumente über an der Kassette angeordnete, auf die Instrumente gerichtete Düsen.

Der im Anspruch 1 angegebenen Erfindung liegt die Aufgabe zugrunde, ein Verfahren und eine Vorrichtung anzugeben, welche zur Intensivreinigung von ärztlichen, insbesondere zahnärztlichen Gegenständen gut geeignet ist. Kennzeichnend für das erfindungsgemäße Verfahren ist, daß in den Impulspausen der Reinigungsflüssigkeit Luft mit höherem Druck pulsierend in die Zuleitung der Reinigungsflüssigkeit zu den Düsen gegeben wird.

Eine nach diesem Verfahren arbeitende Vorrichtung umfaßt eine die Gegenstände aufnehmende Kassette, die in eine druckdicht verschließbare Kammer einlegbar ist, in der der Reinigungsprozeß abläuft. Die Kassette enthält Adapter zur Halterung der Gegenstände. Um die gehalterten Gegenstände herum ist eine Vielzahl von Düsen angeordnet, die gezielt auf die Gegenstände gerichtet sind. Über die Düsen wird Reinigungsflüssigkeit auf die Außenflächen der Gegenstände geleitet.

Es hat sich überraschenderweise gezeigt, daß, wenn man der Reinigungsflüssigkeit in den Impulspausen ein Gas pulsierend zuführt (vorzugsweise mit zeitlich kleineren Impulsen), die kinetische Energie der Flüssigkeit so erhöht wird, daß man mit geringem Wasserverbrauch auch ohne Reinigungsmittel eine sehr hohe Reinigungswirkung erzielen kann. Ein wesentlicher Effekt der Reinigung liegt also darin, daß ein hochenergetischer Wasserstrahl mittels eines dem Wasser stoßweise zugeführten Gases, vorzugsweise mittels Druckluft, erzeugt wird. Die Impulsfrequenz für dieses stoßweise Zuführen des Gases liegt bei etwa 3 Hz.

Das erfindungsgemäße Verfahren sowie eine danach arbeitende Vorrichtung wird nachfolgend anhand der Zeichnung näher erläutert.

Es zeigen:
Figur 1 eine Ausführungsform der erfindungsgemäßen Vorrichtung in einer schaubildlichen Darstellung,
Figur 2 eine Ausführungsform einer in die Vorrichtung nach Figur 1 einsetzbaren Instrumentenkassette in schaubildlicher Explosionsdarstellung,
Figuren 3 und 4 eine Ausführungsform einer Abdeckung an der Rückseite der in Figur 2 gezeigten Kassette,
Figur 5 einen der in Figur 2 gezeigten Adapter und den rückwärtigen Teil eines dazu passenden Instruments im Längsschnitt,
Figur 6 eine vereinfachte Darstellung der Anordnung einer mit Instrument bestückten Kassette im Gerät.
Figur 7 ein hydraulisch/pneumatisches Schaltbild.
Figur 8 ein Impuls-Zeitdiagramm,
Figur 9 eine Variante zu der in Figur 6 gezeigten Ausführungsform.

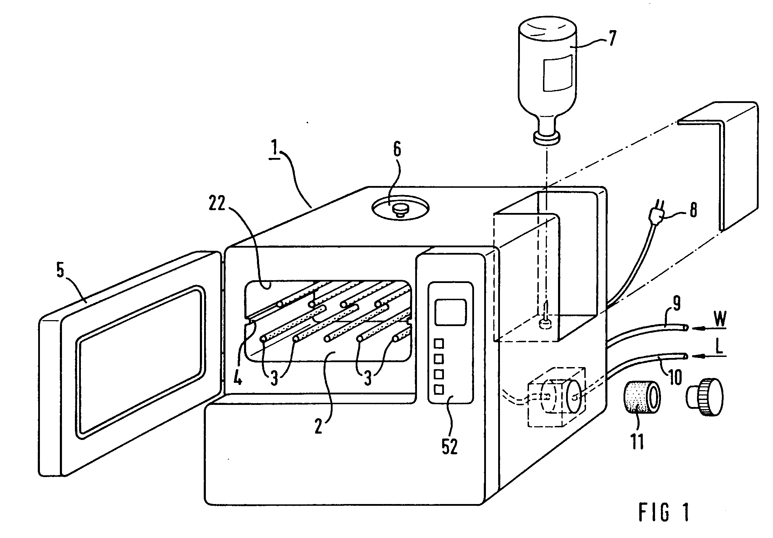

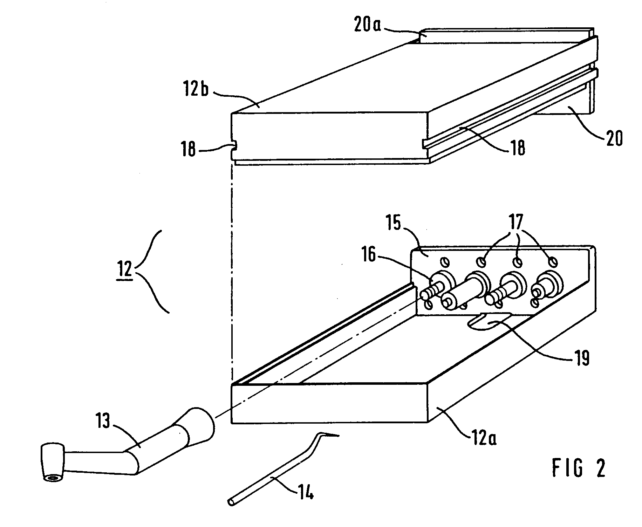

Die Figur 1 zeigt in einer schaubildlichen Darstellung eine Ausführungsform des erfindungsgemäßen Gerätes, welches hier zur hygienischen Aufbereitung diverser zahnmedizinischer Instrumente dient. Das Gerät enthält ein allgemein mit 1 bezeichnetes Gehäuse, welches frontseitig eine Kammer 2 zur Aufnahme einer später noch näher erläuterten Kassette bildet. In die Kammer 2 ragen eine Vielzahl (hier neun) von Düsenarmen 3, mit deren Hilfe, wie ebenfalls später noch näher erläutert, die Instrumente außen besprüht werden können. Beidseitig der Kammer 2 sind Führungsleisten 4 zur Führung der in Figur 2 näher dargestellten Kassette angeordnet. Die Kammer 2 ist in bekannter Weise frontseitig mittels eines Deckels 5 druckdicht verschließbar.

Im rückwärtigen Teil des Gehäuses 1 befindet sich an der Oberseite die Einfüllöffnung 6 eines nicht dargestellten Behälters für demineralisiertes bzw. destilliertes Wasser. Seitlich daneben ist eine abdeckbare Nische zur Aufnahme eines Schmierölvorratsbehälters 7 vogesehen. Dieser Behälter kann in bekannter Weise einen Membranverschluß aufweisen, der beim Aufsetzen auf einen entsprechend ausgebildeten Anschlußzapfen von diesem durchstochen wird, wodurch eine Entnahme des Schmieröls ermöglicht ist. Mit 8, 9 und 10 sind Zuleitungen für elektrische Energie, Druckwasser sowie Druckluft bezeichnet. Mit 11 ist ein leicht entnehmbares und damit austauschbares Sterilfilter bezeichnet, welches im Leitungskanal der Druckluftzuleitung einsetzbar ist. Damit kann sichergestellt werden, daß weitgehend keimfreie Luft für die nachfolgend näher erläuterten Verfahrensschritte zur Verfügung steht.

Die Figur 2 zeigt in einer schaubildlichen Explosionsdarstellung eine in die Kammer 2 einsetzbare Kassette 12 zur Aufnahme mehrerer Instrumente 13 und 14, die hygienisch aufbereitet werden sollen. Das in der Figur mit 13 bezeichnete Instrument ist ein Hand- und Winkelstück, welches im Innern diverse Antriebsteile und Lager sowie Medienkanäle aufweist. Das Instrument 14 dagegen ist ein Handinstrument, welches keine bewegten Teile und auch keine Medienkanäle aufweist.

Die Kassette 12 besteht aus einem Basis- oder Unterteil 12a und einem abnehmbar daran gehalterten Kassettenoberteil 12b. Das Kassettenunterteil 12a ist wannenförmig ausgebildet und enthält an einer erhöhten Rückwand 15 vier verschiedene Adapter zur Aufnahme unterschiedlich gestalteter Instrumente. Im dargestellten Ausführungsbeispiel ist der Adapter 16 auf das aufzunehmende Instrument 13 abgestimmt. Die vier Adapter sind für Instrumente vorgesehen, die bewegte Innenteile und Medienkanäle aufweisen, wie z.B. Hand- und Winkelstücke, Turbinen-, Zahnsteinentfernungs- oder Spritzhandstücke. Instrumente, die keine bewegten Innenteile haben und auch keine Medienkanäle beinhalten, wie beispielsweise das in der Figur mit 14 bezeichnete Handinstrument, werden zur Aufbereitung mittels geeigneter Halterungen am Boden des Kassettenunterteils plaziert.

Die Rückwand 15 des Kassettenunterteils 12a enthält (neun) Bohrungen 17, durch die beim Einsetzen der Kassette 12 in die Kammer 2 die dort vorhandenen (neun) Düsenarme 3 hindurchgreifen können. Die Anordnung der Düsenarme 3 und der mit ihnen korrespondierenden Bohrungen 17 sind, wie aus der Abbildung hervorgeht, jeweils dreieckförmig um die mittig gelegenen Adapter angeordnet.

Das auf das Kassettenunterteil 12a aufsetzbare und mit diesem rastend verbindbare Oberteil 12b enthält seitliche Führungsnuten 18, die mit den bereits erwähnten Führungsleisten (Pos. 4 in Fig. 1) zusammenwirken und eine exakte Zentrierung und Führung der Kassette in der Kammer 2 gewährleisten.

Die Kassette ist an geeigneter Stelle, vorzugsweise bodenseitig und dicht benachbart der Rückwand 15 mit einer oder mehreren Ablauföffnungen 19 für den Ablauf des zugeführten Reinigungs- sowie Kondenswasser versehen.

Vorteilhaftweise ist (sind) diese Ablauföffnung(en) mit einem beim Entnehmen der Kassette selbsttätig verschließenden Teil, z.B. mit einem federbelasteten Schieber oder Deckel, versehen.

Das Kassettenoberteil 12b ist vorteilhafterweise an der Rückseite mit einer Abdeckung versehen sein, welche die Öffnungen 17 und die zu den Adaptern 16 führenden Anschlüsse sowie gegebenenfalls die Ablauföffnung 19 abdeckt bzw. verschließt, wenn die Kassette aus dem Gerät herausgenommen wird.

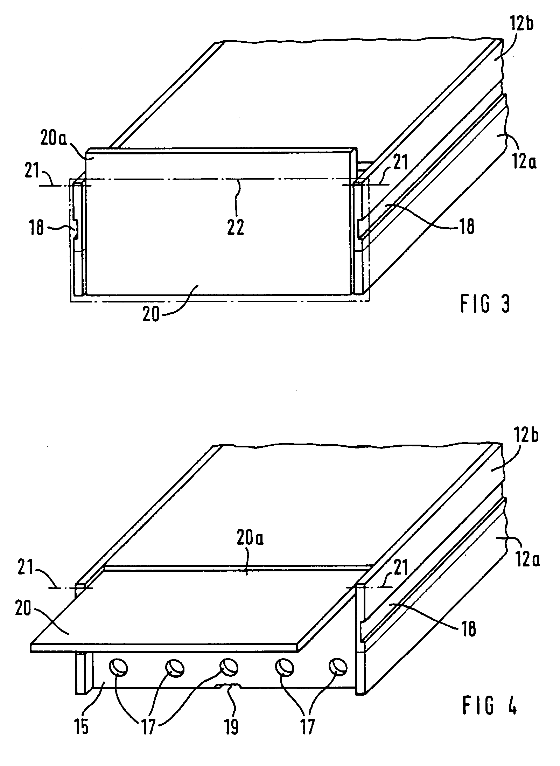

Die Figuren 3 und 4 zeigen eine Ausführungsform einer solchen Abdeckung, wobei die Figuren die Kassette von der Rückseite zeigen. Eine Abdeckplatte 20 ist um ein Lager 21 nach oben aufklappbar. Das Aufklappen geschieht automatisch beim Einschieben der Kassette 12 in die Kammer 2 des Gerätes 1. Hierzu weist die Abdeckplatte 20 einen Überstand 20a auf, der beim Einschieben der Kassette in die Kammer 2 an der mit 22 angedeuteten Oberkante der Kammer 2 anschlägt, wodurch die Klappe nach oben geöffnet wird (Fig. 4). Die sich dahinter befindlichen Öffnungen 17 sowie die aus der Abbildung nicht ersichtlichen Anschlüsse zu den Adaptern 16 werden sodann freigegeben. Beim Herausnehmen der Kassette klappt die Abdeckplatte 20 automatisch wieder nach unten und verschließt so die Öffnungen, so daß die in der Kassette befindlichen, hygienisch aufbereiteten Instrumente gegen Eindringen von Keimen zumindest für einen bestimmten Zeitraum geschützt sind.

Alternativ zu der aufgezeigten, selbsttätig öffnenden und schließenden Klappe kann auch ein Rolladenelement vorgesehen werden, welches mit Hilfe entsprechend vorgesehener Stellmittel eine selbsttätige Abdeckung bzw. Freihaltung der an der Rückwand 15 vorgesehenen Öffnungen bzw. Anschlüsse bewirkt.

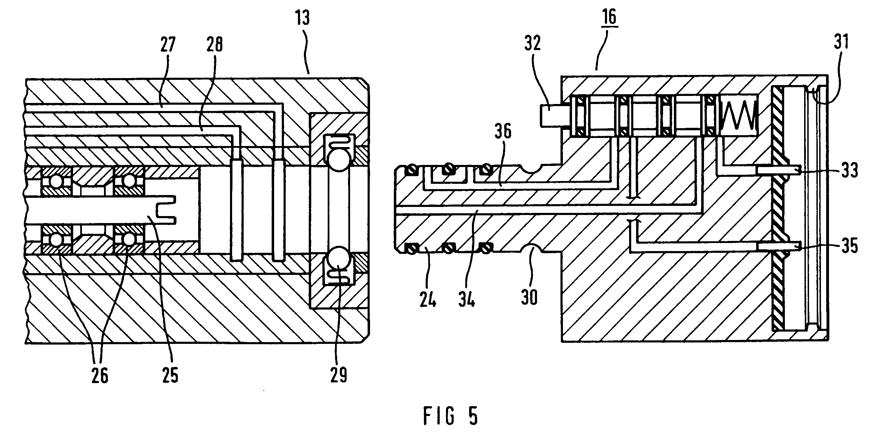

Anhand der Figur 5 wird am Beispiel des Adapters 16 der Aufbau und die Funktion der Adapter sowie das Zusammenwirken mit den daran aufsteckbaren Instrumenten erläutert.

Der Adapter 16 ist hier zum Anschließen eines in Figur 2 mit 13 bezeichneten Hand- und Winkelstückes vorgesehen, welches in seinem Inneren in Rotation versetzbare Triebwellen 25 sowie diesen zugeordnete Lager 26 enthält. Des weiteren verlaufen im Inneren eines solchen Handstückes Medienkanäle 27, 28 zur Zuführung von Kühlluft und Kühlwasser an die Präparationsstelle. Mit 29 ist eine Kugelrasteinrichtung bezeichnet, die beim Aufsetzen des Instruments 13 an einen diesem zugeordneten Antrieb mit einer entsprechenden Ringnut korrespondiert. Eine solche Ringnut ist beim Adapter 16 vorgesehen und dort mit 30 bezeichnet.

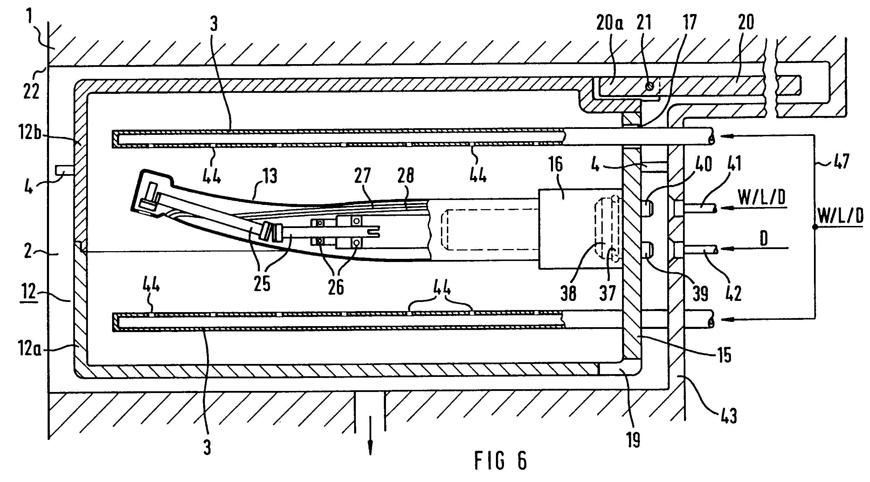

Der Adapter 16 enthält einen Anschlußzapfen 24, der baulich auf die Konstruktion des Instruments abgestimmt ist. An dem dem Instrument abgewandten Ende ist der Adapter 16 so ausgebildet, daß er an der Rückwand 15 des Kassettenunterteils 12a leicht lösbar befestigt werden kann. Hierzu enthält er eine Rastnase 31, die mit einer Ringnut 37 eines Flansches 38 (Fig. 6) an der Rückwand 15 des Kassettenunterteils 12a zusammenwirkt.

Im Adapter 16 befindet sich ein achsparallel verschiebbarer Schaltplunger 32, welcher beim Aufschieben des Instruments 13 entgegen der Kraft einer nicht näher bezeichneten Feder betätigt wird. Bei Betätigung wird der Eingangskanal 33 mit einem zentrisch gelegenen Kanal 34 verbunden. Außerdem wird die Verbindung eines weiteren Eingangskanales 35 mit einem Leitungskanal 36 hergestellt, der im aufgesetzten Zustand des Instruments über periphere Öffnungen mit den Medienleitungen 27, 28 des Instruments verbunden ist. Über den Eingangskanal 33 kann Treibluft oder Schmieröl zu den Triebwellen 25 und deren Lager 26 geleitet werden; über den Leitungskanal 35 kann Luft und Wasser gemeinsam zu den Leitungen 27 und 28 geführt werden.

Mit dem Plungerventil 32 ist sichergestellt, daß bei nicht mit einem Instrument belegtem Adapter die zugeführten Medien am Adapter nicht austreten können.

Wie bereits erwähnt, sind die Anschlußmaße bezüglich der Instrumente für alle vier, in der Kassette gehalterten Adapter unterschiedlich, um so unterschiedliche Instrumente haltern zu können. Die Anschlußmaße bezüglich des Anschlusses an der Rückwand 15 der Kassette sind jedoch für alle Adapter gleich, so daß sie sehr leicht untereinander ausgetauscht bzw. gegen solche mit anderen Anschlußkonturen für andere Instrumente gewechselt werden können.

Anhand der Figur 6 wird am Beispiel des Instruments 13 und des Adapters 16 die Zuordnung der einzelnen Wasch- und Reinigungsdüsen bzw. -kanäle erläutert.

Die Figur 6 zeigt in einer stark vereinfachten Darstellung die Anordnung der Kassette 12 in der Kammer 2 in nahezu vollständig eingeschobenem Zustand. An der Rückwand 15 der Kassette 12 befinden sich, entsprechend der Anzahl der vorhandenen Adapter, Anschlußflansche 38, auf die, wie bereits erwähnt, die Adapter 16 leicht lösbar aufgesetzt werden können. Die Anschlußflansche 38 weisen Verbindungsmuffen 39, 40 auf, welche bei völlig eingeschobener Kassette eine Verbindung herstellen, einerseits zwischen Zuleitungen 41, 42 und den bereits erwähnten Eingangskanälen 33 und 35 (Fig. 5). Die um das Instrument 13 stern- bzw. dreieckförmig herum angeordneten Düsenarme 3 sind an der Gehäusewandung 43, welche die Kammer 2 rückseitig begrenzt, fest angeordnet. Sie sind als Hohlleitungen ausgebildet und weisen eine Vielzahl von Düsenaustrittsöffnungen 44 auf, die unter einem bestimmten Winkel auf die Oberfläche des Instruments 13 ausgerichtet angeordnet sind. Die Düsenbohrungen sind vorteilhafterweise längs der Düsenarme nicht nur in einer Ebene, sondern in mehreren um einen bestimmten Winkel gegeneinander versetzten Ebenen angeordnet. Der Versatz kann vorteilhafterweise zick-zack- oder wellenförmig in Längsrichtung entlang einer Bezugsebene angeordnet sein.

Wie aus Figur 1 und auch aus dem nachfolgend noch näher erläuterten hydraulisch-pneumatischen Schaltplan hervorgeht, sind zur Aufbereitung von maximal vier Instrumenten vorteilhafterweise neun Düsenarme vorgesehen. Die Anordnung ist dabei so getroffen, daß die Düsenarme jeweils in einem Winkel von 120° zueinander um ein Instrument herum angeordnet sind.

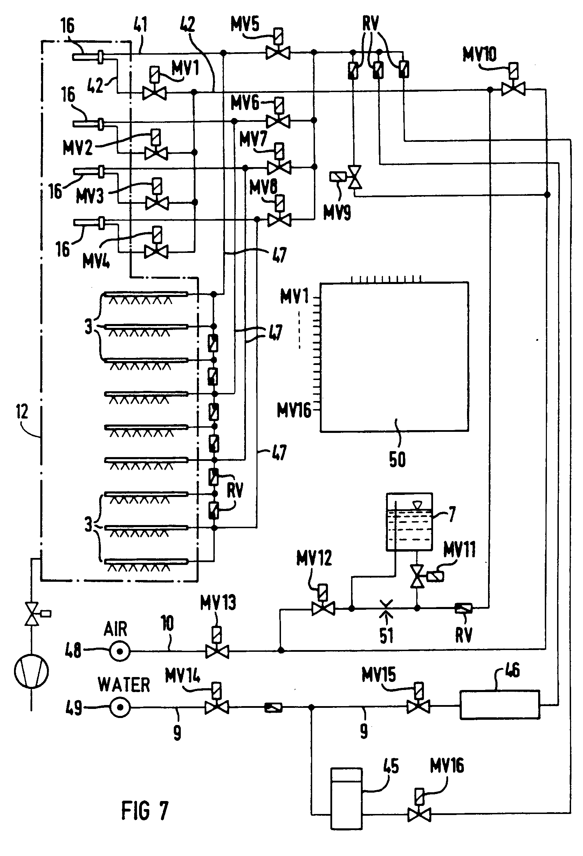

Bevor das erfindungsgemäße Verfahren anhand der bereits erläuterten Zeichnung und des hydraulisch-pneumatischen Blockschaltbildes gemäß Figur 7 näher erläutert wird, sei noch erwähnt, daß im Gerätegehäuse außer dem bereits erwähnten Ölbehälter 7 auch ein an die Druckwasserleitung 9 angeschlossener Dampferzeuger 45 sowie eine Vorwärmeinrichtung 46 für Wasser untergebracht sind.

Die Figur 7 zeigt ein hydraulisch-pneumatisches Schaltbild von der erfindungsgemäßen Einrichtung.

Mit 48 und 49 sind die Versorgungsquellen bezeichnet, mit denen einerseits Druckluft und andererseits Druckwasser in das Gerät eingespeist werden. Die eingangsseitig üblichen Filter, Ölabscheider (bei Druckluft) sowie Überdruckventile sind der Einfachheit halber nicht eingetragen.

Sämtliche Magnetventile (MV1 bis MV16) sowie die Wasserheizung 46 und der Dampferzeuger 45 werden von einem Mikroprozessor 50 aus gesteuert. Mit RV sind in die Leitungen geschaltete Rückschlagventile bezeichnet.

Die Injektion des Schmiermittels (mit Luft vermischbares Pflegeöl) aus dem Behälter 7 erfolgt hier pneumatisch über die beiden Magnetventile MV1 und MV 12 sowie eine Droselstelle 51. Alternativ kann das Schmieröl auch mittels einer geeigneten Ölpumpe zugeführt werden. Der MP 50 steuert die Magnetventile MV1 bis MV9 und MV11 bis MV16 so, daß den Adaptern 16 und den Düsenarmen 3 die Medien Luft, Wasser und gegebenenfalls das Schmieröl bei den Verfahrensschritten der Vor- und der Nachreinigung - und gegebenenfalls beim Pflegevorgang - für jedes Instrument getrennt zugeführt werden, so daß die Instrumente zeitlich nacheinander gereinigt - und gegebenenfalls gepflegt - werden.

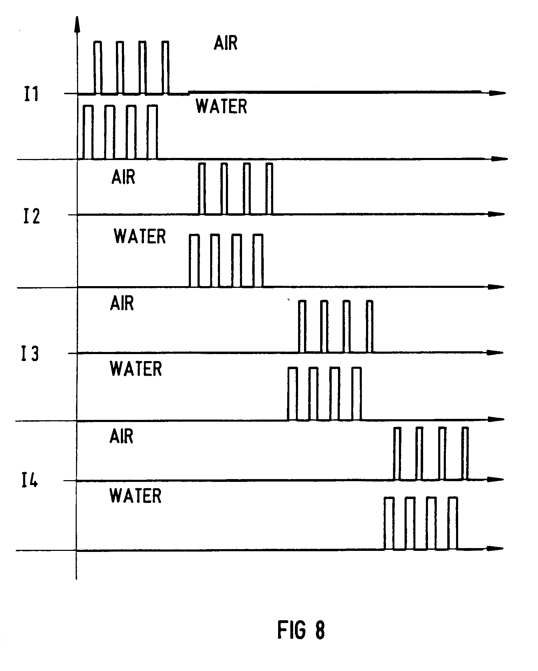

Aus Figur 8, die den zeitlichen Verlauf der Ventilanschaltung für die Medien Luft und Wasser für vier Instrumente (im Diagramm mit I1 bis I4 bezeichnet) aufzeigt, ist dieser zeitliche Versatz erkennbar. Mit dieser zeitlich aufeinanderfolgenden Zuschaltung der Medien wird bei niedrigem Wasserverbrauch eine hohe Reinigungseffizienz erzielt.

Das erfindungsgemäße Verfahren läuft wie folgt ab:
Zunächst wird die Kassette 12 mit Instrumenten beschickt. Instrumente, die bewegbare Innenteile haben, wie Winkelstücke, Turbinen, Spritzen, od.dgl., werden auf die entsprechenden Adapter 16 (max. vier) der Kassette aufgesteckt. Die übrigen Instrumente werden an Haltevorrichtungen über dem Behälterboden plaziert. Die Kassette wird sodann durch Aufsetzen des Kassettenoberteils verschlossen und in die Kammer 2 des Gerätes eingeführt. Mit dem Einführen wird die rückwärtige Abdeckung 20 angehoben, wodurch die neun Düsenarme 3 durch die Bohrungen 17 der Kassettenrückwand hindurchgreifen können. Nachdem die Kassette in der Kammer 2 so weit eingeschoben ist, daß die Verbindung der Leitungen 41, 42 mit den Anschlüssen 39, 40 (Fig. 6) hergestellt ist, wird der Deckel 5 das Gerät geschlossen und das Gerät eingeschaltet. Nach einem Selbsttest, der etwa eine halbe Minute dauert, erfolgt zunächst eine Kaltreinigung der Instrumente, indem über die Zuleitung 9 und die Leitungen 41 und 47 den Adaptern 16 und den Düsenarmen 3 kaltes Wasser zugeführt wird. Das kalte Wasser wird vorzugsweise pulsierend zugeführt, wobei dem Wasser in den Impulspausen Druckluft mit höherem Druck (ebenfalls pulsierend) beigemischt wird (Fig. 8). Dadurch wird ein hochenergetischer Wasserstrahl erzeugt, der überraschenderweise eine sehr gründliche Reinigung der Oberfläche und auch der Innenkanäle bewirkt. Gleichzeitig wird über die Leitung 42 der Getriebekanal mit Luft beaufschlagt (das Ölzuführungsventil MV11 bleibt geschlossen), wodurch das Eindringen von Schmutz und (verschmutztem) Reinigungswasser verhindert wird. Die Impulsfrequenz, mit der dem Wasser Druckluft zugeführt wird, beträgt etwa 3 Hz.

Die Kaltreinigung ist nach etwa 2 Minuten beendet. Danach erfolgt eine Warmreinigung der Außenflächen der Instrumente und auch der Innenkanäle, ebenfalls mit einem pulsierenden Wasserstrahl, unter Beifügung von gepulster Druckluft. Dieser Vorgang läuft wie vorerwähnt ab. Nach etwa 2 Minuten Warmreinigung erfolgt eine intensive Nachreinigung und Dampfdesinfektion durch Ab-und Ausblasen der Außenflächen der Instrumente und der Medien-und Getriebekanäle im Innern der Instrumente mit Heißwasser von 60° bis 100°C. Das Wasser wird hierzu von dem als Durchlauferhitzer wirkenden Erhitzer 46 erwärmt. Dieser Vorgang dauert etwa 30 bis 45 Sekunden.

Nach dieser intensiven Nachreinigung erfolgt eine Pflege der bewegten Innenteile durch Injektion einer dosierten Menge Schmieröl aus dem Schmierölvorratsbehälter 7. Die Zudosierung kann in Abhängigkeit vom adaptierten Instrument erfolgen und beträgt im Mittel 1 Gramm pro Instrument und Injektionszyklus.

Dieser Pflegeschritt dauert etwa eine halbe Minute. Anschließend erfolgt eine Sterilisation der Instrumente, und zwar sowohl von außen als auch von innen, mittels gesättigtem Wasserdampf von vorzugsweise 134°C bei einem Druck von etwa 2 bis 2,5 bar. Der Sterilisationsvorgang beträgt zwischen 5 und 6 Minuten. Danach folgt ein Trocknen und Abkühlen der Instrumente mittels kalter Luft. Der Abkühlvorgang dauert etwa 3 bis 5 Minuten. Bei einer Gesamtzeit von etwa 15 bis 20 Minuten zur Aufbereitung der Instrumente kann danach die Kassette mit den aufbereiteten Instrumenten entnommen und im geschlossenen Zustand zur Benutzung bereitgestellt werden.

Der Verfahrensablauf, der an sich selbsttätig in der geschilderten Weise abläuft, kann durch Eingriff, z.B. durch entsprechende Programmvorwahl, die an einem Bedien- und Displayfeld 52 an der Frontseite des Gerätes abgerufen werden kann, variiert werden, z.B. indem im Anschluß an die intensive Nachreinigung und Desinfektion der Außenfläche eine Zwischentrocknung, z.B. durch Luft, durchgeführt wird. Ebenso kann nach dem Pflegen eine Unterbrechung stattfinden, um so nicht sterilisierbare Gegenstände aus der Kassette entnehmen zu können.

Zur Entfernung vorhandener Trockenluft, insbesondere aus Hohlräumen, und im besonderen aus Hohlräumen der Instrumente, kann gemäß einer vorteilhaften Variante die Kammer vor dem Sterilisieren der Instrumente evakuiert werden. Um eine optimale Entfernung der Trockenluft zu erzielen, sollte das Vakuum im Bereich zwischen 40 und 500 mbar absolut liegen. Das Vakuum kann nach dem an sich bekannten fraktionierten (mehrstufigen) Verfahren oder nach dem Vorvakuumverfahren erzeugt werden.

Mit dem geschilderten Verfahren ergeben sich extrem kurze Zeiten für eine optimale hygienische Aufbereitung der Instrumente. Durch den pulsierenden Wasserstrahl, dem stoßweise Druckluft höherer Energie zugeführt wird, ist eine besonders intensive Reinigung der Teile gewährleistet. Dadurch, daß nach der Aufbereitung die Instrumente in der Kassette verbleiben können, ist ein sicherer Transport zum Behandlungsplatz gegeben. Das gleiche gilt für den Weg nach Benutzung der Instrumente zur Aufbereitung. Die nach der Benutzung mit Keimen kontaminierten Instrumente verbleiben bis zur Wiederentnahme in der Kassette, wodurch u.a. eine Verletzungsgefahr für das Bedienpersonal ausgeschlossen ist. Bearbeitung, Lagerung und Transport erfolgen somit in einer Kassette. Nachdem die Kassette weitgehend abgeschlossen ist, ist eine sterile Lagerung der Instrumente zumindest über einen hinreichend langen Zeitraum (Stunden) gewährleistet. Dadurch, daß die Innenteile und die Medienkanäle mit Heißwasser und Dampf durchströmt werden, ergibt sich eine relativ rasche Aufheizzeit der gesamten Instrumente. Durch das pulsierende Ab- und Ausblasen der Teile mit Wasserdampf wird auch ein gewisser kalklösender Effekt in den Leitungen erzielt. Ein wesentlicher Vorteil ist, daß keinerlei Reinigungs- und Treibmittel verwendet zu werden brauchen.

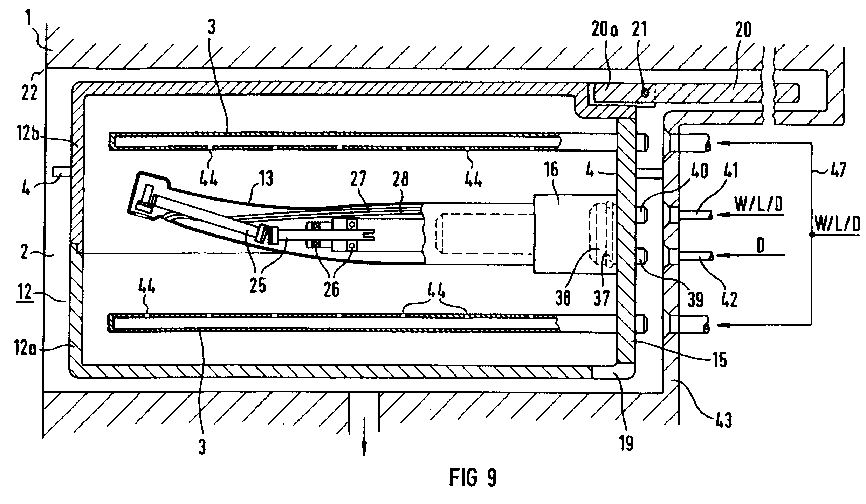

Hinsichtlich der Anordnung und Ausbildung der Düsenarme 3 sind im Rahmen der Erfindung verschiedene Modifikationen denkbar. So können beispielsweise die Düsenarme 3 auch Bestandteil der Kassette sein, wie dies im Ausführungsbeispiel nach Fig. 9 dargestellt ist. Des weiteren könnten, obgleich dies relativ aufwendig wäre, die Düsenarme um ihre Längsachse schwenkbar und gegebenenfalls auch in Achsrichtung bewegbar angeordnet sein. Die in den Fig. 3 bis 5 dargestellte Abdeckung kann alternativ auch Bestandteil des Kassettenunterteils sein oder beiden Kassettenhälften zugeordnet sein.

## Patentansprüche

1. Verfahren Zur Intensivreinigung von ärztlichen, insbesondere zahnärztlichen Instrumenten und gegebenenfalls in deren Anwendungsbereich fallenden anderen Gegenständen mit Hilfe einer unter Druck zugeführten und über Düsen (44) auf die Instrumente bzw. Gegenstände gerichtet austretenden Reinigungsflüssigkeit,
**dadurch gekennzeichnet,** daß die Reinigungsflüssigkeit pulsierend zugeführt wird und in den Impulspausen der Flüssigkeitszufuhr Luft unter höherem Druck pulsierend in die Zuleitung (47) der Reinigungsflüssigkeit zu den Düsen (44) gegeben wird.

2. Vorrichtung zur Intensivreinigung von ärztlichen, insbesondere zahnärztlichen Instrumenten und ggf. in deren Anwendungsbereich fallenden anderen Gegenständen, enthaltend eine die Gegenstände (13, 14) aufnehmende Kassette (12), die in eine druckdicht verschließbare Kammer (2) einlegbar ist, wobei die Kassette Adapter (16) zur Halterung der Gegenstände enthält, dadurch gekennzeichnet, daß die Kassette (12) eine Vielzahl von auf die Instrumente gerichteten Düsen (44) enthält, die um die gehalterten Gegenstände herum angeordnet sind, über die die Reinigungsflüssigkeit zur Behandlung der Außenflächen der Gegenstände zuführbar ist, und daß eine Steuereinrichtung (50) vorhanden ist, welche die Zufuhr einerseits von Luft und andererseits von Reinigungsflüssigkeit so steuert, daß die Reinigungsflüssigkeit pulsierend zugeführt wird und in den Impulspausen der Flüssigkeitszufuhr Luft unter höherem Druck pulsierend in die Zuleitung (47) der Reinigungsflüssigkeit zu den Düsen (44) gegeben wird.

3. Vorrichtung nach Anspruch 2, bei der zumindest für Gegenstände (13), die Medienkanäle (27, 28) und bewegte Innenteile (25) aufweisen die Adapter (16) Mittel (24) zur Halterung der gegenstände (13) sowie Mittel (33, 35) zur Zufuhr von fluiden und gasförmigen Medien zur Behandlung der bewegten Innenteile (25) und der Medienkanäle (27, 28) beinhalten.

4. Vorrichtung nach Anspruch 2 oder 3,
bei der die Düsen (44) an Teilen der Kammer (2) angeordnet sind und die Kassette (12) mit Öffnungen für den Zu- und Ablauf der Behandlungsmedien versehen ist.

5. Vorrichtung nach Anspruch 2 oder 3, bei der die Düsen (44) an Teilen der Kassette (12) angeordnet sind und die Kassette mit Öffnungen für den Zu- und Ablauf der Behandlungsmedien versehen ist.

6. Vorrichtung nach Anspruch 4, bei der die Düsen (44) an Düsenarmen (3) angeordnet sind, die sich entlang der Kammer (2) erstrecken und am rückwärtigen Ende der Kammer (2) befestigt und so angeordnet sind, daß die Oberflächen der Gegenstände vollständig mit den Behandlungsmedien beaufschlagbar sind, wobei eine Rückwand (15) der Kassette (12) mit Öffnungen (17) versehen ist, durch die beim Einschieben der Kassette in die Kammer (2) die Düsenarme (3) hindurchgreifen.

7. Vorrichtung nach Anspruch 4 oder 5, bei der sich die Düsen (44) an Düsenarmen (3) befinden, die in einem Winkel von 120° zueinander um die Gegenstände angeordnet sind.

8. Vorrichtung nach Anspruch 7,
bei der die Düsen (44) entlang der Düsenarme (3), bezogen auf eine durch die Längsachse verlaufende Bezugsebene, gegeneinander um einen kleinen Winkel versetzt angeordnet sind.

9. Vorrichtung nach Anspruch 8,
bei der der Versatz so ausgerichtet ist, daß die Düsen (44) alternierend ober- und unterhalb der Bezugsebene verlaufen.

10. Vorrichtung nach einem der Ansprüche 6 bis 9,
bei der die Düsenarme (3) um die Gegenstände (13) herum so angeordnet sind, daß für einen Adapter (16) die Düsen (44) von drei Düsenarmen (3) wirksam sind.

11. Vorrichtung nach Anspruch 10,
bei der für zwei benachbarte Adapter (16) mindestens ein gemeinsamer Düsenarm (3) vorgesehen ist.

12. Vorrichtung nach einem der Ansprüche 2 bis 11,
bei der die Adapter (16) leicht wechselbar an der Rückwand (15) der Kassette (12) gehaltert sind.

13. Vorrichtung nach Anspruch 12,
bei der mindestens ein Adapter (16) zur Halterung von einem zahnärztlichen Instrument (13) vorgesehen ist, welches Leitungskanäle (27, 28) zur Zuführung der Behandlungsmedien in das Innere des Instruments aufweist.

14. Vorrichtung nach Anspruch 13,
bei der der Adapter (16) ein Steuerventil (32) beinhaltet, welches die Zuführungsleitungen (33, 35) bei nicht mit einem Instrument (13) belegtem Adapter (16) verschließt.

15. Vorrichtung nach Anspruch 13,
bei der mehrere Adapter (16) für unterschiedliche Instrumente (13) vorgesehen sind, wobei die Adapter bezüglich ihrer Halterung an der Gehäusewand (15) gleiche Anschlußmaße aufweisen.

16. Vorrichtung nach einem der Ansprüche 2 bis 15,
bei der die Kassette (12) aus zwei durch Querteilung gebildeten und miteinander verbindbaren Gehäusehälften (12a, 12b) besteht, wobei die bodenseitige Gehäusehälfte (12a) den/die Adapter (16) trägt und die deckseitige Gehäusehälfte (12b) Führungselemente (4) zur Führung der Kassette (12) in der Kammer (2) des Gerätes aufweist.

17. Vorrichtung nach einem der Ansprüche 6 bis 11,
bei der die Öffnungen (17) in der Kassette (12) für den Durchtritt der Düsenarme (3) von einem Abdeckmittel (20) verschlossen werden, wobei dem Abdeckmittel ein Steuermittel (20a) zur Freigabe der Öffnungen zugeordnet ist.

18. Vorrichtung nach Anspruch 17,
bei der das Abdeckmittel eine an der Rückwand (15) der Kassette (12) schwenkbar gehalterte Platte (20) ist, die einen Überstand (20a) aufweist, der beim Einführen der Kassette in die Kammer (2) an einer Gehäusekante (22) zu Anlage kommt und dabei die Platte im Sinne einer Freigabe der Öffnungen (17) verstellt.

19. Vorrichtung nach einem der Ansprüche 2 bis 18,
bei der die Kassette (12) aus einem sterilisierbaren Kunststoff besteht.

20. Vorrichtung nach Anspruch 2, bei der die Steuereinrichtung (50) so ausgelegt ist, daß die Impulsfrequenz für das stoßweise Zuführen des Gases etwa 3 Hz beträgt.

## Claims

1. Process for the intensive cleaning of medical, in particular dental, instruments, and other articles which may fall within the area of application thereof, with the aid of a cleaning fluid which is supplied under pressure and is discharged via nozzles (44) in a manner in which it is directed onto the instruments or articles, characterized in that the cleaning fluid is supplied with pulsing action and, in the pauses between pulses of the liquid supply, air is introduced with pulsing action, at higher pressure, into the supply line (47) of the cleaning fluid to the nozzles (44).

2. Apparatus for the intensive cleaning of medical, in particular dental, instruments, and other articles which may fall within the area of application thereof, containing a module (12) which receives the articles (13, 14) and can be positioned in a chamber (2) which can be closed off in a pressure-tight manner, the module containing adapters (16) for securing the articles, characterized in that the module (12) contains a multiplicity of nozzles (44) which are directed onto the instruments, are arranged around the secured articles and via which it is possible to supply the cleaning fluid for treating the outer surfaces of the articles, and in that there is provided a control device (50) which controls the supply of air, on the one hand, and of cleaning fluid, on the other hand, such that the cleaning fluid is supplied with pulsing action and, in the pauses between pulses of the fluid supply, air is introduced with pulsing action, at higher pressure, into the supply line (47) of the cleaning fluid to the nozzles (44).

3. Apparatus according to Claim 2, in the case of which, at least for articles (13) which have media-carrying ducts (27, 28) and moving inner parts (25), the adapters (16) contain means (24) for securing the articles (13) and means (33, 35) for supplying fluid and gaseous media for treating the moving inner parts (25) and the media-carrying ducts (27, 28).

4. Apparatus according to Claims 2 or 3, in the case of which the nozzles (44) are arranged on parts of the chamber (2), and the module (12) is provided with openings for the supply and discharge of the treatment media.

5. Apparatus according to Claims 2 or 3, in the case of which the nozzles (44) are arranged on parts of the module (12), and the module is provided with openings for the supply and discharge of the treatment media.

6. Apparatus according to Claim 4, in the case of which the nozzles (44) are arranged on nozzle arms (3) which extend along the chamber (2) and are fastened, and arranged, at the rear end of the chamber (2) such that the surfaces of the articles are subjected to the action of the treatment media to the full extent, a rear wall (15) of the module (12) being provided with openings (17) through which the nozzle arms (3) engage when the module is pushed into the chamber (2).

7. Apparatus according to Claims 4 or 5, in the case of which the nozzles (44) are located on nozzle arms (3) which are arranged at an angle of 120° with respect to one another around the articles.

8. Apparatus according to Claim 7, in the case of which the nozzles (44) are arranged along the nozzle arms (3) such that they are offset by a small angle with respect to one another in relation to a reference plane running through the longitudinal axis.

9. Apparatus according to Claim 8, in the case of which the offset positioning is such that the nozzles (44) run in an alternating manner above and beneath the reference plane.

10. Apparatus according to one of Claims 6 to 9, in the case of which the nozzle arms (3) are arranged around the articles (13) such that the nozzles (44) of three nozzle arms (3) are active for one adapter (16).

11. Apparatus according to Claim 10, in the case of which at least one common nozzle arm (3) is provided for two adjacent adapters (16).

12. Apparatus according to one of Claims 2 to 11, in the case of which the adapter (16) is secured on the rear wall (15) of the module (12) such that it can easily be exchanged.

13. Apparatus according to Claim 12, in the case of which at least one adapter (16) is provided for securing a dental instrument (13) which has line ducts (27, 28) for supplying the treatment media into the interior of the instrument.

14. Apparatus according to Claim 13, in the case of which the adapter (16) contains a control valve (32) which closes off the supply lines (33, 35) when the adapter (16) is not occupied by an instrument (13).

15. Apparatus according to Claim 13, in the case of which a plurality of adapters (16) are provided for different instruments (13), the adapters having the same connection dimensions as far as being secured on the housing wall (15) is concerned.

16. Apparatus according to one of Claims 2 to 15, in the case of which the module (12) comprises two housing halves (12a, 12b) which are formed by being divided transversely and can be connected to one another, the base-side housing half (12a) bearing the adapter(s) (16), and the top-side housing half (12b) having guide elements (4) for guiding the module (12) in the chamber (2) of the unit.

17. Apparatus according to one of Claims 6 to 11, in the case of which the openings (17) in the module (12) for the through-passage of the nozzle arms (3) are closed off by a covering means (20), the covering means being assigned a control means (20a) for releasing the openings.

18. Apparatus according to Claim 17, in the case of which the covering means is a plate (20) which is secured pivotably on the rear wall (15) of the module (12) and has a projection (20a) which, when the module is introduced into the chamber (2), comes into abutment against a housing edge (22) and thus adjusts the plate with the effect of releasing the openings (17).

19. Apparatus according to one of Claims 2 to 18, in the case of which the module (12) consists of a sterilizable plastic.

20. Apparatus according to Claim 2, in the case of which the control device (50) is designed such that the pulse frequency for the pulsating supply of the gas is approximately 3 Hz.

## Revendications

1. Procédé pour le nettoyage intensif d'instruments médicaux, notamment d'instruments de dentisterie et éventuellement d'autres objets entrant dans le domaine d'utilisation de ces instruments, à l'aide d'un liquide de nettoyage envoyé sous pression et sortant par l'intermédiaire de buses (44) en étant dirigé sur les instruments ou les objets, caractérisé en ce que le liquide de nettoyage est envoyé d'une manière pulsatoire, et pendant des pauses entre les impulsions d'envoi du liquide, de l'air est envoyé d'une manière pulsatoire, sous une pression accrue, dans la canalisation (47) d'amenée du liquide de nettoyage aux buses (44).

2. Dispositif pour le nettoyage intensif d'instruments médicaux, notamment d'instruments de dentisterie et éventuellement d'autres objets entrant dans le domaine d'utilisation de ces instruments, comportant une cassette (12), qui reçoit les objets (13,14) et qui peut être insérée dans une chambre (2) pouvant être fermée d'une manière étanche à la pression, la cassette contenant des adaptateurs (16) pour le maintien des objets, caractérisé en ce que la cassette (12) contient une multiplicité de buses (44) dirigées vers les instruments et qui sont disposées autour des objets retenus et au moyen desquelles le liquide de nettoyage peut être amené pour le traitement des faces extérieures des objets, et qu'il est prévu un dispositif de commande (50), qui commande l'envoi d'une part d'air et d'autre part d'un liquide de nettoyage de telle sorte que le liquide de nettoyage est envoyé d'une manière pulsatoire et que pendant les pauses entre les impulsions d'envoi de liquide, de l'air sous une pression accrue est envoyé de façon pulsatoire dans la canalisation (47) d'amenée du liquide de nettoyage aux buses (44).

3. Dispositif selon la revendication 2, dans lequel au moins pour des objets (13), qui comportent des canaux (27,28) pour des milieux et des parties intérieures mobiles déplacées (25), les adaptateurs (16) contiennent des moyens (24) pour retenir les objets (13) ainsi que des moyens (33,35) pour envoyer des milieux fluides et gazeux pour le traitement des parties intérieures mobiles (25) et des canaux (27,28) pour les milieux.

4. Dispositif selon la revendication 2 ou 3, dans lequel les buses (44) sont disposées sur des parties de la chambre (2) et que la cassette (12) comporte des ouvertures pour l'amenée et la sortie des milieux de traitement.

5. Dispositif selon la revendication 2 ou 3, dans lequel les buses (44) sont disposées sur des parties de la cassette (12) et la cassette est pourvue d'ouvertures pour l'amenée et l'évacuation des milieux de traitement.

6. Dispositif selon la revendication 4, dans lequel les buses (44) sont disposés sur des bras (3) de support de buses, qui s'étendent le long de la chambre (2) et sont fixés sur l'extrémité arrière de la chambre (2) et sont disposés de telle sorte que les surfaces des objets peuvent être chargées complètement par les milieux de traitement, une paroi arrière (15) de la cassette (12) étant pourvue d'ouvertures (17), dans lesquelles s'engagent les bras porte-buses (3) lors de l'insertion de la cassette dans la chambre (2).

7. Dispositif selon la revendication 3 ou 4, dans lequel les buses (44) sont situées sur des bras porte-buses (3), qui sont disposés en faisant un angle de 120° entre eux autour des objets.

8. Dispositif selon la revendication 7, dans lequel les buses (44) sont disposées par rapport à un plan de référence passant par l'axe longitudinal, en étant décalées réciproquement d'un angle faible.

9. Dispositif selon la revendication 8, dans lequel le décalage est aligné de telle sorte que les buses (44) s'étendent alternativement au-dessus et au-dessous du plan de référence.

10. Dispositif selon l'une des revendications 6 à 9, dans lequel les bras porte-buses (3) sont disposés autour des objets (13) de telle sorte que les buses (44) de trois bras porte-buses (3) sont actives pour un adaptateur (16).

11. Dispositif selon la revendication 10, dans lequel au moins un bras porte-buses commun (3) est prévu pour deux adaptateurs (16) voisins.

12. Dispositif selon l'une des revendications 2 à 11, dans lequel les adaptateurs (16) sont retenus de manière à être aisément interchangeables sur la paroi arrière (15) de la cassette (12).

13. Dispositif selon la revendication 12, dans lequel au moins un adaptateur (16) est prévu pour le maintien d'un instrument de dentisterie (13), qui comporte les canaux formant conduites (27,28) pour l'amenée des milieux de traitement à l'intérieur de l'instrument.

14. Dispositif selon la revendication 13, dans lequel l'adaptateur (16) contient une soupape de commande (32), qui ferme les conduites d'amenée (33,35) lorsque l'adaptateur (16) ne contient aucun instrument (13).

15. Dispositif selon la revendication 13, dans lequel plusieurs adaptateurs (16) sont prévus pour différents instruments (13), les adaptateurs possédant les mêmes cotes de raccordement que leur support sur la paroi (15) du boîtier.

16. Dispositif selon l'une des revendications 2 à 15, dans lequel la cassette (12) est constituée de deux moitiés de boîtier (12a,12b) qui sont formées par une subdivision transversale et peuvent être reliées entre elles, la moitié de boîtier (12a) située du côté du sol portant le/les adaptateurs (16), et la moitié de boîtier (12b) située du côté du plafond comportant des éléments de guidage (4) servant à guider la cassette (12) dans la chambre (2) de l'appareil.

17. Dispositif selon l'une des revendications 6 à 11, dans lequel les ouvertures (17) formées dans la cassette (12) pour le passage des bras porte-buses (3) sont formées par un moyen de recouvrement (20), un moyen de commande (20a) servant à libérer les ouvertures étant associé au moyen de recouvrement.

18. Dispositif selon la revendication 17, dans lequel le moyen de recouvrement est une plaque (20) qui est retenue de manière à pouvoir pivoter sur la paroi arrière (15) de la cassette (12) et qui possède une partie en saillie (20a), qui lors de l'insertion de la cassette dans la chambre (2) vient s'appliquer contre un bord (22) du boîtier et déplace la plaque dans le sens d'une libération des ouvertures (17).

19. Dispositif selon l'une des revendications 2 à 18, dans lequel la cassette (12) est réalisée en une matière plastique pouvant être stérilisée.

20. Dispositif selon la revendication 2, dans lequel le dispositif de commande (50) est agencé de telle sorte que la fréquence des impulsions pour l'amenée impulsionnelle du gaz est égale à environ 3 Hz.
